# EUROPEAN PATENT APPLICATION

(11) **EP 4 764 646 A1**
(43) Date of publication of application: **24.06.2026**
(21) Application number: 24221204.1
(22) Date of filing: 18.12.2024
(51) Int. Cl.: G02B 21/00, G02B 21/36

(54) **DEVICE AND METHOD FOR AN AUTOMATIC FIELD-OF-VIEW CONTROL DURING A SURGICAL PROCEDURE**

(71) Applicant: Leica Instruments (Singapore) Pte Ltd, Singapore 618299 (SG)
(72) Inventor: Jung, David, 618299 Singapore (SG)
(74) Representative: Paustian & Partner Patentanwälte mbB

(57) **Abstract**

A first aspect of the present disclosure is related to a device for an automatic control of a field-of-view, FOV, during a surgical procedure,
configured to:
- obtain an image of a sample by a movable imaging device , wherein the image is captured from a first perspective ;
- identify the sample within the image;
- identify an obstacle within the image;
- provide a control information for the imaging device to move the imaging device to capture the sample from a second perspective , which is different from the first perspective.

## Description

### Technical Field

This disclosure is related to devices for automatic control of a field-of-view during a surgical procedure and to methods therefor.

### Background

Imaging during surgery can be difficult when obstacles like instruments cover or obscure a view on a sample. Obstacles may also scatter or block light, reducing clarity and contrast. During a surgical procedure, a surgeon may be unable or unwilling to release their instrument to manually adjust position or orientation of the microscope to optimize the field of view. As a result, the sample may not be fully visible in the microscope's image. Improvement are therefore desirable.

### Summary

An object of the present disclosure is to improve imaging of a sample.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a device for an automatic control of a field-of-view, FOV, during a surgical procedure,
*configured to:*
- *obtain an image of a sample by a movable imaging device* , *wherein the image is captured from a first perspective* ;
- *identify the sample within the image*;
- *identify an obstacle within the image*;
- *provide a control information for the imaging device to move the imaging device to capture the sample from a second perspective* , *which is different from the first perspective.*

The device according to the first aspect can be a control device, e.g. a specially programmed computer, configured to control the imaging device and/or it can be a part of the movable imaging device.

An obtaining of an image in the sense of this disclosure can comprise can receiving and/or a fetching of this information. Additionally or alternatively, this can comprise determining this information based on other received/fetched information and/or based on a means for capturing an image.

A sample can be any object that can be viewed with an imaging device for and/or during a surgical procedure. It can be an organ of a patient and/or a part of an organ. It can be a biopsy, in particular one that is isolated and analyzed in situ. A sample can be or comprise a bone and/or blood of a patient. A sample can also comprise a suture, an incision, or any other artifact placed within or at a being's body for or during a surgical procedure.

A movable imaging device can be a versatile optical instrument designed with flexible workspace by the help of joints and/or an adjustable elements. Thereby, it may allow for precise positioning and/or easy movement of the imaging device's optical system. This may enable users to analyze specimen at different positions within a workspace that is larger than the focal range of the imaging device. This mobility may enable users to manipulate the imaging device to view specimens from various angles without needing to move the specimen itself. Additionally or alternatively, such a mobility may allow users to analyze a plurality of specimen and/or a larger specimen at different positions. Additionally or alternatively, such a mobility allows users to operate the imaging device in one or more working positions (in which the device is used for imaging specimen) and other positions, in which the device is, e.g., parked during a longer operation and/or a series of imaging tasks. In this way an imaging device allows for a shared workspace with a user. Therefore, movable imaging devices are particularly useful in fields that require adaptability and ergonomic convenience, e.g., such as medical diagnostics and/or laboratory settings. The movable design facilitates greater accessibility to hard-to-reach areas, ensuring detailed examination of samples with minimal disruption. Additionally, this flexibility may enhance user comfort by allowing the imaging device to be adjusted to the user's posture, reducing strain during extended periods of observation. A movable imaging device can be any camera-based tool that provides a (magnified) view of an observation object, such as a movable microscope, a movable exoscope, a movable endoscope, and/or a movable surgical camera.

A perspective refers to the viewpoint from which the imaging device captures a scene/sample. A perspective may be influenced by a position, an orientation, and an lens properties of the imaging system. It determines how objects appear in terms of size, depth, and spatial relationships. Perspective shapes how a three-dimensional scene projects onto the camera's two-dimensional image plane.

Identification of a sample in an image may involve analyzing its physical, chemical, and structural characteristics with one or more techniques. Visual inspection may be a first step, focusing on morphology, texture, and other observable features, often compared with reference samples or guides. Depending on the sample type, staining or fluorescent labeling may have been applied to enhance visibility or highlight specific components. Therefore, such labels may also be identified for identifying a sample. Additionally or alternatively identification of a pre-defined spectrum in the image may indicate a sample. Additionally or alternatively an identification may comprise a pattern recognition within the image, in particular based on a statistical method and/or machine learning. Additionally or alternatively, an identification of a sample may be based on an identification of an obstacle, e.g., such that everything which is not identified as an obstacle may be identified as a sample.

An identifying of an obstacle within an image can be based on the same or similar methods and will further be explained in more detail below. In particular a plurality of obstacles can be identified, in particular a further obstacle can be identified, when a second perspective is realized.

A providing of a control information in the sense of this disclosure can comprise sending the information and/or storing the information for others to fetch, e.g. storing the information in a shared memory. Based on the control information, the movable imaging device can move or be moved to a perspective from which the obstacle is less disturbing for the image, e.g. the second perspective can be a perspective in which the view of the sample by the image is fully undisturbed by the view. Additionally or alternatively, a second perspective can be chosen in which the sample is (fully) visible albeit the obstacle is also visible. In general, based on the control information, a FOV of a surgeon or any other personal can be adapted to mitigate the influence of an obstacle that at least partly prevents a direct view of a sample.

An embodiment of the first aspect is related to a device for an automatic control of a field-of-view, FOV, during a surgical procedure,
*configured to:*
- *determine based on the image a degree to which the image is impaired by the obstacle*;
- *determine the control information such that the second perspective yields a lesser degree of impairment than the first perspective* .

A degree of impairment can be determined based on different metrics. A signal-to-noise Ratio (SNR) may be used to determine the ratio of the target signal to noise introduced by the obstacle. Contrast reduction may be used to evaluate how an obstacle diminishes the contrast between the target and its background. Metrics based on a modulation transfer function may be used to measure the devices ability to reproduce spatial frequencies despite the obstacle's presence. Blur and sharpness metrics, e.g. Laplacian variance, may be used to assess how an obstacle reduces image clarity or causes blurring. Furthermore, it may be quantified the extent to which the target is obscured, using techniques like histogram analysis, edge detection, or occlusion measurement. Additionally or alternatively, a sample area covered by an obstacle and/or an image area covered by an obstacle can be determined, in particular quantified. One or more of these metrics may be configured based on empirical information, i.e. based on a user-perception.

Based on a degree of impairment, control information can be provided to not just change an obstacle-impaired perspective, but to change the perspective such that an impairment of a user view of the sample due to the obstacle is improved. Furthermore, a control information can be issued to optimize the FOV by minimizing a degree of impairment.

An embodiment of the first aspect is related to a device for an automatic control of a field-of-view, FOV, during a surgical procedure,
*configured to identify the sample manually, in particular by one or more of the following:*
- *a manual focus adjustment;*
- *a selection of a part of a sample in the obtained image by a computer-based pointing device.*

Manual identification of a sample in an image may involves visually examining the image and marking or labeling one or more interesting parts (i.e. the sample(s)) using annotation tools. Thereby, a sample can be manually identified by using bounding boxes, polygons, freehand outlines, or point markers. In particular different, non-connected and/or difficult to recognize parts of a sample can be identified manually. A computer-based pointing device can be, e.g., a mouse, a trackpad, and/or an eyetracker.

An embodiment of the first aspect is related to a device for an automatic control of a field-of-view, FOV, during a surgical procedure,
*configured to identify the sample automatically*, *in particular by one or more of the following:*
- *an object identification;*
- *an identification of a predefined imaging parameter.*

Automatic identification of one or more samples (or parts of a sample) in an image may rely on computer algorithms, e.g. object recognition and/or machine learning techniques. An automatic identification may start with preprocessing an image to enhance relevant features, such as resizing, noise reduction, and/or contrast adjustment. Additionally or alternatively, feature extraction may be performed, using descriptors like edges, textures, or advanced methods such as, e.g., a scale-invariant feature transform, a histogram of oriented gradients, and/or deep learning feature maps.

Additionally or alternatively, object detection may be applied. An object detection may comprise template matching and/or contour detection. Furthermore, machine learning models or deep learning frameworks may be used to identify a sample and differentiate it from foreground and/or background of the image that does not show the sample. Machine-learning-based identification may be performed using neural networks like ResNet or VGG. Additionally, an automatic identification (but also a manual identification) may comprise a postprocessing to refine the identification result. This may eliminate false positives, and outputs are stored with bounding boxes, labels, and confidence scores for further analysis or visualization, in particular for a distinction between sample and obstacle.

An embodiment of the first aspect is related to a device for an automatic control of a field-of-view, FOV, during a surgical procedure,
*configured to identify the obstacle based on a color of an area in the image.*

To identify an obstacle based on color in an image, the image may be converted to a suitable color space, such as HSV or LAB, which simplifies color-based segmentation. A target color range may be defined, specifying the hue, saturation, and brightness values. A binary mask may then be created, highlighting pixels within this range. The identified obstacle can then be analyzed further by finding contours, calculating properties, or performing classification tasks, in particular by machine learning methods.

An embodiment of the first aspect is related to a device for an automatic control of a field-of-view, FOV, during a surgical procedure,
*configured to identify the obstacle based on a focus value of an area in the image.*

Identifying an obstacle based on a focus value in an image may comprise analyzing a sharpness and/or a clarity of different regions. Usually a sample may be in focus and may exhibit distinct edges and higher-frequency content, whereas an obstacle may be out-of-focus exhibiting less distinct edges and lower-frequency content.

For an identification based on focus value, sharpness metrics may be computed across the image. One method may involve calculating a variance of the Laplacian, where a high variance indicates sharp focus, while a low variance suggests blur. Another approach may be to analyze a gradient magnitude, which measures edge intensities, as sharper areas have stronger gradients. Alternatively, a wavelet transform may be used to isolate low-frequency components associated with obstacle regions.

A focus map may be determined by dividing the image into smaller regions and/or by applying a sliding window to determine a sharpness value for each section. Thresholding the focus map isolates regions with low sharpness, and additionally operations can be applied to refine these regions, e.g. by removing noise.

An obstacle may be identified by segmenting the areas with the low/lowest focus values. These areas can then be a starting point for providing further techniques to identify other parts of an obstacle that are depicted with higher focus values. This can be analyzed by finding contours, calculating properties, or performing classification tasks, in particular by machine learning methods.

An embodiment of the first aspect is related to a device for an automatic control of a field-of-view, FOV, during a surgical procedure,
*configured to identify the obstacle based on a form and*/*or a location of a consistent area in the image.*

Finding an obstacle (or part(s) of an obstacle) within an image based on its form and location may be done by analyzing its shape, spatial properties and/or its relationship to other elements in the image. The process may involve identifying consistent patterns and/or regions that match pre-defined characteristics.

Therefore, a preprocessing may be performed to enhance the image and facilitate the detection of consistent areas. This may include converting the image to grayscale, applying filters to reduce noise, and/or enhancing edges. Shape-based analysis may comprise edge detection, which can highlight contours and/or boundaries in the image. Contours or connected components may then be extracted.

Once contours or areas are identified, they may be analyzed for specific geometric properties such as aspect ratio, circularity, and/or symmetry, depending on the obstacles known form. Location-based criteria, such as proximity to a predefined region or alignment with other objects, may further narrow the search or lead to an identification of the most deteriorating parts of an obstacle. For example, objects in the center of the frame or near known landmarks can be prioritized.

Consistent areas that match a form and/or a location may be segmented for further processing or analysis. These areas can then be a starting point for providing further techniques to identify other parts of the obstacle that had not been identified based on form and/or location.

An embodiment of the first aspect is related to a device for an automatic control of a field-of-view, FOV, during a surgical procedure,
*configured to identify the obstacle based on a rate of change of a consistent area in the image.*

To find an obstacle within an image based on the rate of change may require at least a second image for determining a rate of change, in particular an image stream. The process may begin with preprocessing the images to enhance features, normalize resolution, and/or reduce noise. Background subtraction or frame differencing helps isolate moving objects. Regions of interest may be detected using methods like edge detection or contour extraction, focusing on their geometric properties, such as shape and size.

To identify movement of an obstacle or of a part of an obstacle, algorithms like optical flow, Kalman filters, and/or deep-learning trackers may be used to estimate motion. Additionally or alternatively, consistency of the identified obstacles across images may be provided by assigning unique IDs to obstacles. A rate of change may be measured by determining variations in position, size, or shape, such as shifts in centroid coordinates, bounding boxes, and/or aspect ratios. Objects showing significant changes (e.g. changes over a pre-defined threshold) in position, velocity, deformation, and/or trajectory maybe flagged as obstacles, in particular if the sample can be considered as only slow moving or not moving at all.

An embodiment of the first aspect is related to a device for an automatic control of a field-of-view, FOV, during a surgical procedure, *configured to identify the obstacle based on an object recognition.*

Object recognition may be used be to identify and/or locate an obstacle in an image by analyzing its features and matching it to predefined categories. The process may comprise feature extraction, where algorithms or models analyze key attributes such as edges, textures, and patterns to represent objects mathematically. Machine learning techniques, such as neural networks, may be used to learn hierarchical features automatically.

A trained object recognition model may be applied to detect and classify objects in the image. These models may analyze features to predict object categories and their spatial locations and may be configured to output bounding boxes with confidence scores. The results are filtered to prioritize objects meeting specific criteria, such as category, location, and/or confidence level to identify them as obstacles or part of an obstacle. The identified areas can then be used as starting point for further techniques to identify other parts of the obstacle that had not been identified based on the object recognition.

An embodiment of the first aspect is related to a device for an automatic control of a field-of-view, FOV, during a surgical procedure,
*configured to identify the obstacle based on a plurality of images captured by the imaging device.*

Obstacles in an image can be identified by analyzing consecutive images to detect changes or disruptions in the scene, or by capture 3D properties which can then in particular be recognized by an object recognition. A background model may be built to highlight deviations caused by obstacles, using methods like frame differencing or Gaussian Mixture Models. Additionally or alternatively, motion analysis through optical flow detects interruptions in movement patterns, while feature tracking identifies stationary elements inconsistent with the background. Additionally or alternatively depth information from disparity maps (in case of stereo images) may reveal obstacles through irregularities in scene geometry (The static part of an image may be qualified as sample and the dynamic part of an image may be identified as obstacle.

The different embodiments for obstacle identification can be combined in part or altogether to improve the obstacle identification.

An embodiment of the first aspect is related to a device for an automatic control of a field-of-view, FOV, during a surgical procedure,
*configured to:*
- *display to a user of the imaging device if an obstacle has been identified and*/*or that a second perspective is available; and*
- *obtain information from a user interface that the second perspective should be taken.*

A user-based confirmation of a movement to a second perspective may enhance safety and also efficacy. Safety is increased by giving confirmation to the movable imaging device before a movement is started and thereby preventing a surprising action. Furthermore, the device will not disturb any surgical personal by a movement to a second perspective when this movement is not desired, e.g. because a current degree of impairment by an obstacle may be too small. A confirmation can be provided by a haptic input, by audio input (voice command), and/or by a gesture.

An embodiment of the first aspect is related to a device for an automatic control of a field-of-view, FOV, during a surgical procedure,
*configured to:*
   - *continuously determine the obstacle* ;
   - *continuously provide control information for a second perspective* ;
   - *stop an adjustment to a second perspective if one or more of the following*
*conditions is fulfilled:*
   -- a *degree of impairment of the view of the sample by the obstacle is below a pre-defined threshold;*
   -- *a second obstacle is identified.*

A continuous determination of an obstacle can be determined, e.g., based on every image of an image stream or based on every sample image of an image stream, wherein a sample may be captured every 2nd, 5th, 10th, 20th image or once within a pre-defined time period, such as every 5s, 30s, every minute or the like.

In a first mode, control information can be provided to move the imaging device into a second perspective and stay there. In a second mode, control information can be provided to move the imaging device into the second perspective if a certain condition is fulfilled. This condition can be a degree of impairment by a (dynamic) obstacle, being larger than a pre.-defined threshold. The imaging device can than remain there or adapt to further second perspectives. And in case the obstacle has vanished (or at least the degree of impairment has dropped below a pre-defined threshold), control information can be such that the imaging device then automatically moves back to a first perspective. In this case, the first perspective may be regarded or defined as a standard or a prioritized perspective. Advantageously, adaptation to a second perspective can be stopped if a FOV on the sample is sufficient, or if adaptation would not improve a FOV on a sample.

In general, image-based control for a movable imaging device, may also be called visual servoing, and may be based on real-time visual feedback to guide the imaging device's movements. It may comprise processing the images to extract sample and obstacle information such as position, orientation, or contours of the sample/obstacle, and using this information to compute control commands. The process may be provided by defining a desired visual state, such as aligning the Imaging device with the sample. Sample and/or obstacle features are tracked, in particular continuously or quasi-continuously, and their deviations from the desired state are computed. These deviations are converted into control signals using a visual servoing algorithm, often involving a Jacobian matrix to relate changes in image features to the control motions of the imaging device. Based on the control signals a second perspective is adjusted. The imaging device may be controlled to adjust its position or orientation iteratively until the desired second perspective or a further second perspective is realized.

An embodiment of the first aspect is related to a device for an automatic control of a field-of-view, FOV, during a surgical procedure,
*configured to:*
- *use obstacle identification to reposition the imaging device according to a user preference.*

By this function a user can use the device to reposition the imaging device, in particular according to her/his specific needs. In order to switch in a repositioning mode, the device may have to identify a specific obstacle, e.g., a hand of a user, by which the user preference is expressed. Then a translational movement can be commanded and/or a rotation. This can be defined by an additional information, such as the form of the identified human hand and or an additional command, such as a haptic and/or an audio input. For example by pointing with a pre-defined finger in a certain direction, the device can identify in which direction the movable imaging device should be moved and issue control information correspondingly. Thereby, a user can reposition the imaging device by using the obstacle avoidance.

An embodiment of the first aspect is related to a device for an automatic control of a field-of-view, FOV, during a surgical procedure,
*configured to:*
*- determine based on the image a degree to which the image is impaired by an obstacle;*
*- consider the obstacle only, if the degree of impairment is larger than* a *pre-defined threshold.*

The device can ignore impairments of a FOV by an obstacle if the impairment is smaller than a pre-defined threshold and/or if based on a statistical and/or machine learning algorithm it is recognized that a user view has sufficient quality for a certain operation.

A second aspect of the present disclosure is related to a method for an automatic control of a field-of-view, FOV, during a surgical procedure,
*comprising the steps:*
*- obtaining an image of a sample by an movable imaging device* , *wherein the image is captured from a first perspective* ;
*- identifying the sample within the image;*
*- identifying an obstacle within the image;*
*- providing a control information for the imaging device to move the imaging device to capture the sample from a second perspective* , *which is different from the first perspective.*

A method according to the second aspect can comprise steps that implement one or more functions and/or features as described in context with the first embodiment.

A further aspect of the present disclosure is related to a computing device comprising a processor configured to carry out the method according to any one of preceding aspects/embodiments.

A further process of the present disclosure is related to a computer program product comprising instructions which, when the program is executed by a computer system, cause the computer system to carry out the method according to any one of the preceding aspects/embodiments.

A further aspect of the present disclosure is related to a computer-readable medium comprising instructions which, when executed by a computer system, cause the computer system to carry out the method according to any one of the preceding aspects/embodiments.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a control of a movable imaging device according to an embodiments of this disclosure.
Fig. 2 illustrates a method for controlling an imaging device according to an embodiment of this disclosure.
Fig. 3 illustrates a microscope system according to or for embodiments of this disclosure.

Although some aspects have been described in the context of an apparatus (or a system) in the present disclosure, the description of these aspects also represents a description of the corresponding method, where a block or device corresponds to a method step or a feature of a method step.

Analogously, aspects described in the context of a method step also represent a description of a corresponding block, item, or feature of a corresponding apparatus or of a system that may in particular be distributed over different locations and is configured to exchange information between the different locations with respective communication means.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the aspects or embodiments of this disclosure, until explicitly stated otherwise.

In the following description reference is made to the accompanying figures which form part of the disclosure, and which illustrate specific aspects in which the present disclosure can be understood. Identical reference signs refer to identical or at least functionally or structurally similar features.

### Detailed description

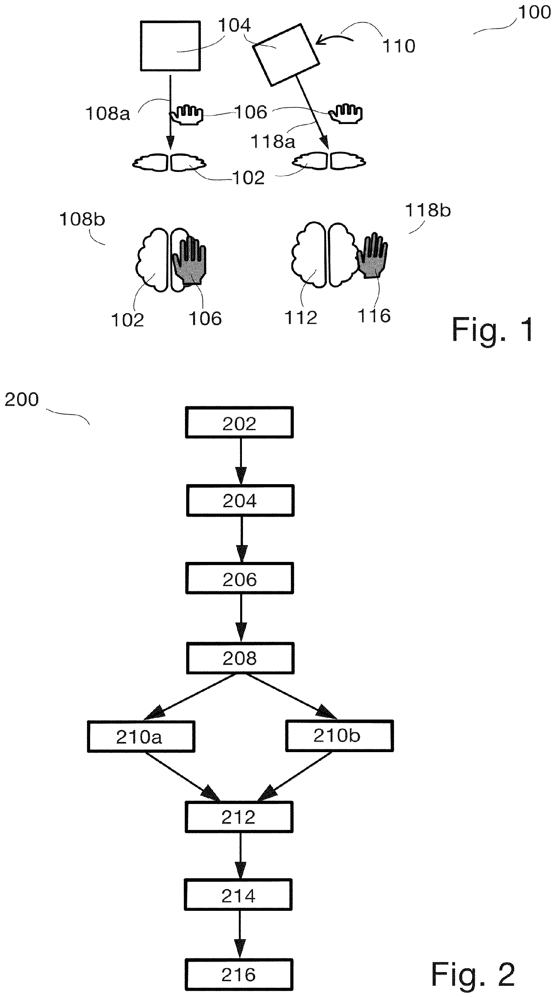

Fig. 1 illustrates an operation of a device 100 for an automatic control of a field-of-view, FOV, during a surgical procedure, according to an embodiment of this disclosure. Only the lens system 104 of the movable imaging device is shown.

In the left part of the figure, movable imaging device observes a sample 102 from a first perspective 108a. The FOV 108b from the first perspective 108a shows that the sample 102 can only be captured partly because it is covered by an obstacle 106. To improve this situation, the obstacle 106 is identified. The obstacle can be identified by different methods that may operate, e.g., on parameters such as color, focus value, location, area, and/or rate of change of certain parts of the image. Based on this, the sample 102 is identified as the remainder of the image, which does not belong to the obstacle. Based on the kinematics of imaging device and their relation to the FOV of image (i.e. model of imaging device and environment/sample) a second perspective 118a is determined. Control information is then determined and issued to the controller of the imaging device 104 by which the imaging device changes its position 110 such that the second perspective 118a is realized. This is illustrated at the right part of the figure. The FOV 118b from the second perspective 118a provides a nearly free view of the sample 112, which is not impaired by the obstacle 116 anymore.

However, the view 112 of the sample from the second perspective maybe undesirable because the sample is, e.g., distorted in perspective. Therefore, the position of the obstacle may be tracked also when the movable imaging device is in a second perspective 118a. In case it is determined that the obstacle 106, 116 has vanished or at least moved further out of the FOV determined by the first perspective, than control information can be sent to the controller of the movable imaging device 104 to return to the first perspective.

Additionally or alternatively, the obstacle avoidance can be used to control the position of movable imaging device. Therefore, the obstacle must be determined as a specific obstacle based on which a position-based control of the imaging device 104 is performed. In the illustrated situation this is indicated by the obstacle 106 being a human's hand. After recognizing the specific obstacle, the device switches into a continuous re-adjustment mode in which new second perspectives are commanded to the imaging device as long as the hand is determined as being in the FOV of the device. The mode can be terminated by an audio command (or a gesture) from a human user such that the imaging device remains in the position after the continuous repositioning mode was ended. In a specific embodiment, by moving the hand into the field of view the virtual optical axis of the imaging device, the device can be pushed in the desired direction. Either a rotation or a linear movement could be performed depending on preferences and settings. In case of this active control the imaging device may react quickly to the hand in the field of view while in the avoidance mode (mode in which the imaging device should not be actively controlled by a specifically determined obstacle), the imaging may only move after the hand has blocked the view for a pre-defined time period.

Fig. 2 shows a flow chart 200 of a method according to an embodiment of this disclosure. The embodiment consists of two main parts. In a first part an identification of a user's hand and/or of another obstacle in the FOV of an image by a movable imaging device and a differentiation to an object-to-be-observed (sample) is performed. In a second part, a movement of a movable microscope is controlled to a position that optimizes the FOV with respect to a sample, i.e. in which the hand is less visible, while not moving further than necessary.

At the beginning, the microscope observes a sample from a first perspective. A sample identification and an obstacle identification are operating on one or more images obtained from the microscope's camera. During the first part, an obstacle is detected 202. The differentiation between sample and obstacle obstructing the FOV can be done based on several parameters. One or more colors may be different in parts of the obstacle compared to parts of the tissue. Hence, an image of an obstacle may be likely to comprise fewer red tones and/or be grey in case of tools and/or blue in case of surgical gloves. Image areas of an obstacle may be out of focus as not inside the surgical cavity (sample). This may result in less features (lower spatial frequencies), which can also be used as indicator. Only large obstacle areas may need to be avoided, areas which are more than 5%, 10%, 20%, or 50% of the image. Furthermore, in difference to a sample and obstacle may be moving. This can also be a differentiator. A 3D-mapping of the scene (e.g. a disparity map) may also be possible in order to separate a foreground (obstacle) from a background (sample).

After the obstacle has been detected a timer is started 204. Afterwards it is determined if the obstacle impairs a FOV of the microscope longer than a threshold time 206. If this is the case, a kinematics of the microscope (e.g. an articulated arm) is moved (second part of the method) to a second perspective 208 in which an improved FOV with respect to the sample is obtained. For avoiding an obstacle two modes are realized in this embodiment. In a first mode, the microscope moves automatically to obtain a second perspective with a full view of a surgical cavity. In a second mode, a prompt is presented to the user stating that a better perspective on the surgical cavity (sample) is possible and ask whether the microscope should be moved in that direction.

Once the obstructing object is removed, the microscope can be moved back to the original position to restore the original view. Alternatively, the new position can be kept. Again, automatic movement in particular together with a user prompt-based approval of a movement can be realized.

Concurrently to the movement of the microscope, the obstacle identification can still be operating, such that a second obstacle can be identified 210a. Additionally or alternatively, it can be identified that the first obstacle is out of a FOV of the microscope 21 0b. In either of these cases a control information can be issued to stop the movement of the microscope 212. The microscope movement can be stopped when either the obstructing object is not visible anymore (or reduced to a predefined area or degree of impairment) or another object (e.g. the second hand of the surgeon) appears on the other side of the image. Further, in case it is determined that the first obstacle has been removed (e.g. by the obstacle identification, or by a user input) 214, control information can be issued to return the microscope to its first perspective again 216.

Some embodiments relate to a microscope comprising a system as described in connection with one or more of the Figs. 1 to 2. Alternatively, a microscope may be part of or connected to a system as described in connection with one or more of the Figs. 1 to 2.

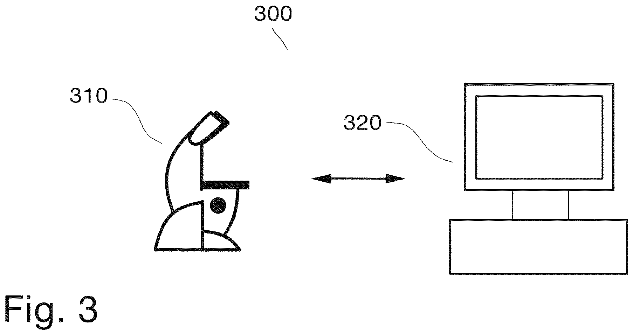

Fig. 3 shows a schematic illustration of a system 300 configured to perform a method described herein. The system 300 comprises a microscope 310 and a computer system 320. The microscope 310 is configured to take images and is connected to the computer system 320. The computer system 320 is configured to execute at least a part of a method described herein. The computer system 320 may be configured to execute a machine learning algorithm. The computer system 320 and microscope 310 may be separate entities but can also be integrated together in one common housing. The computer system 320 may be part of a central processing system of the microscope 310 and/or the computer system 320 may be part of a subcomponent of the microscope 310, such as a sensor, an actor, a camera or an illumination unit, etc. of the microscope 310.

The computer system 320 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference signs

- 100: control of movable imaging device
- 102: sample
- 104: optical system of imaging device
- 106: obstacle
- 108a: first perspective
- 108b: FOV of first perspective
- 110: change to second perspective
- 112: sample of second perspective
- 116: obstacle from second perspective
- 118a: second perspective
- 118b: FOV of second perspective
- 200: flow chart of method
- 202: obstacle detected
- 204: start timer
- 206: obstacle in view for defined duration
- 208: move microscope
- 210a: new obstacle in view at opposite site
- 210b: obstacle out of view
- 212: stop movement
- 214: original object removed
- 216: move back to original position
- 300: microscope system
- 310: microscope
- 320: computer

## Claims

1. A device for an automatic control of a field-of-view, FOV, during a surgical procedure,
configured to:
- obtain an image of a sample (102) by a movable imaging device (104), wherein the image is captured from a first perspective (108a, 108b);
- identify the sample within the image;
- identify an obstacle (106) within the image;
- provide a control information (110) for the imaging device to move the imaging device to capture the sample from a second perspective (118a, 118b), which is different from the first perspective.

2. The device according to the preceding claim,
configured to:
- determine based on the image a degree to which the image is impaired by the obstacle (106);
- determine the control information (110) such that the second perspective (118a, 118b) yields a lesser degree of impairment than the first perspective (108a, 108b).

3. The device according to one of the preceding claims,
configured to identify the sample (102) manually, in particular by one or more of the following:
- a manual focus adjustment;
- a selection of a part of a sample in the obtained image by a computer-based pointing device.

4. The device according to one of the preceding claims,
configured to identify the sample (102) automatically, in particular by one or more of the following:
- an object identification;
- an identification of a predefined imaging parameter.

5. The device according to one of the preceding claims,
configured to identify the obstacle (106) based on a color of an area in the image.

6. The device according to one of the preceding claims,
configured to identify the obstacle (106) based on a focus value of an area in the image.

7. The device according to one of the preceding claims,
configured to identify the obstacle (106) based on a form and/or a location of a consistent area in the image.

8. The device according to one of the preceding claims,
configured to identify the obstacle (106) based on a rate of change of a consistent area in the image.

9. The device according to one of the preceding claims,
configured to identify the obstacle (106) based on an object recognition.

10. The device according to one of the preceding claims,
configured to identify the obstacle (106) based on a plurality of images captured by the imaging device.

11. The device according to one of the preceding claims,
configured to:
- display to a user of the imaging device if an obstacle (106) has been identified and/or that a second perspective is available; and
- obtain information from a user interface that the second perspective (118a, 118b) should be taken.

12. The device according to one of the preceding claims,
configured to:
- continuously determine the obstacle (106);
- continuously provide control information for a second perspective (118a, 118b) ;
- stop an adjustment to a second perspective (118a, 118b) if one or more of the following conditions is fulfilled:
-- a degree of impairment of the view of the sample by the obstacle (106) is below a pre-defined threshold;
-- a second obstacle is identified.

13. The device according to one of the preceding claims,
configured to:
- use obstacle (106) identification to reposition the imaging device according to a user preference.

14. The device according to one of the preceding claims,
configured to:
- determine based on the image a degree to which the image is impaired by an obstacle (106);
- consider the obstacle (106) only, if the degree of impairment is larger than a pre-defined threshold.

15. A method for an automatic control of a field-of-view, FOV, during a surgical procedure,
comprising the steps:
- obtaining an image (102) of a sample by an movable imaging device (104), wherein the image is captured from a first perspective (108a, 108b) ;
- identifying the sample within the image;
- identifying an obstacle (106) within the image;
- providing a control information (110) for the imaging device to move the imaging device to capture the sample from a second perspective (118a, 118b), which is different from the first perspective.
